# EUROPÄISCHE PATENTANMELDUNG

(11) **EP 1 870 073 A1**
(43) Veröffentlichungstag der Anmeldung: **26.12.2007**
(21) Anmeldenummer: 06012980.6
(22) Anmeldetag: 23.06.2006
(51) Int. Cl.: A61K 6/06

(54) **Material und Rohling für Zahnersatz**

(71) Anmelder: Aepsilon Rechteverwaltungs GmbH, 82166 Grägelfing (DE)
(72) Erfinder: Holzner, Stephan, 80269 Hohenschäftlam (DE); Weber, Gerhard, 82266 Inning am Ammersee (DE)
(74) Vertreter: Grünecker, Kinkeldey, Stockmair & Schwanhäusser Anwaltssozietät

(57) **Zusammenfassung**

Die Erfindung betrifft ein Material für Zahnersatz, das eine 3-Punkt-Biegefestigkeit von mindestens 1300 MPa aufweist. Weiterhin betrifft die Erfindung ein aus einem solchen Material hergestellten Zahnersatz sowie entsprechende Rohlings-Materialien und Verfahren zum Herstellen von Rohlingen. Auch betrifft die Erfindung ein Verfahren zum Herstellen von einem Zahnersatzteil aus einem solchen Material oder einem Rohling.

## Beschreibung

Die Erfindung betrifft Ausgangsstoffe und Rohlinge für die Herstellung von Zahnersatzteilen.

Zahnersatzteile können mittels automatisierter Herstellungsmethoden aus Rohmaterialien wie Keramiken oder Metallen gefertigt werden. Hierbei werden die Zahnersatzteile z. B. aus Vollmaterialien gefräst. Im Falle von keramischen Materialien werden die gefrästen Zahnersatzteile in der Regel dicht gesintert, um so Zahnersatzteile mit einer hohen Bruchfestigkeit zu erhalten.

Aufgabe der vorliegenden Erfindung ist es, die Ausgangsstoffe von Zahnersatzteilen, Rohlinge und dazugehörige Verfahren und damit auch die Zahnersatzteile zu verbessern.

Diese Aufgabe wird gelöst durch ein Material nach Anspruch 1, ein Zahnersatzteil nach Anspruch 6, ein Material nach Anspruch 7 oder 8, einen Rohling nach den Ansprüchen 12 oder 13 sowie durch einen Satz vom Rohling nach Anspruch 19, ein Verfahren zum Herstellen von Rohlingskörpern oder Rohlingen nach Anspruch 20 und ein Verfahren zum Herstellen von einem Zahnersatzteil nach Anspruch 31.

Bevorzugte Ausführungsformen sind in den abhängigen Ansprüchen offenbart.

Durch aufwendige Untersuchungen hat sich herausgestellt, dass es möglich ist, Material für einen Zahnersatz zu schaffen, das eine 3-Punkt-Biegefestigkeit von mindestens 1300 MPa hat. Das Material ist bevorzugterweise ein keramisches Material wie etwa Zirkonoxid oder umfasst Zirkonoxid. Statt Zirkonoxid wird für das selbe Material oft auch der Begriff Zirkoniumdioxid verwendet.

Sämtliche Angaben über eine 3-Punkt-Biegefestigkeit in diesem Dokument beziehen sich auf die in der Norm ISO 6872 definierte 3-Punkt-Biegefestigkeit.

Vorteilhaft ist weiterhin ein Material, das eine 3-Punkt-Biegefestigkeit von mindestens 1400, 1500, 1600, 1700, 1800, 1900 oder 2000 MPa aufweist. Je höher die 3-Punkt-Biegefestigkeit ist, desto höher ist auch die Belastbarkeit von Zahnersatzteilen, die aus diesem Material hergestellt wurden, so dass Zahnersatzteile mit dünneren Wandstärken oder solche, die größeren Belastungen standhalten, möglich werden.

Weiterhin ist es vorteilhaft, wenn das Material Dichtschwankungen von weniger als 10%, 5% oder 2% aufweist. Derartige Dichtschwankungen führen zu einer inhomogenen Druckfestigkeit, so dass es vorkommen kann, dass ungünstigerweise genau an einer hoch belasteten Stelle des Zahnersatzteils das Material nur eine geringe Dichte und somit eine geringe Bruchfestigkeit aufweist.

Das Material kann weiterhin einen hohen Zirkonoxid sowie zumindest einen gewissen Yttriumoxidanteil aufweisen. Weiter kann Hafniumoxid hinzugesetzt werden und/oder ein Metalloxid oder Metallsalz.

Das Material kann beispielsweise einen Zirkonoxidanteil über 90, 92, 94, 96 oder 97 % aufweisen, jedoch nicht mehr als 92, 94, 96, 98 oder 99 %. Hafniumoxid kann gar nicht oder mit mindestens 0,5, 1, 1,5, 2, 2,5, 3 % und/oder mit nicht mehr als 0,5, 1, 1,5, 2, 2,5, 3, 3,5, 4 % vorliegen. Yttriumoxid liegt mit einem Anteil von mehr als 1,5, 2, 2,5, 3, 3,5, 4, 4,5, 5 % und/oder mit einem Anteil von nicht höher als 2, 2,5, 3, 3,5, 4, 4, 5, 5, 5, 5, 6 % vor.

Weiterhin kann mit einem Anteil von mindestens 0,01, 0,02, 0,05, 0,1, 0,2, 0,3, 0,4, 0,5 % und/oder mit einem Anteil von nicht mehr als 0,1, 0,2, 0,3, 0,4, 0,5, 0,6, 0,7, 0,75 % eines oder mehre der Oxide von Aluminium, Gallium, Germanium, Indium, Zinn, Blei, Lanthaniten, Metallen, Eisen und/oder einem oder mehreren Metallsalzen vorliegen.

Die in diesem Dokument verwendeten Prozentangaben der chemischen Zusammensetzung beziehen sich auf Gewichtsprozent.

Die letzteren Oxide oder Salze sind bei der Herstellung des Materials möglichst homogen verteilt worden, so dass sich Kristallisationskeime und damit auch Kristallitgrößen möglichst homogen ergeben. Weiterhin können hierdurch die Kristallisationsachsenausrichtungen beeinflusst werden und zwar dahingehend, dass sie möglichst gleichmäßig in alle Raumrichtungen verteilt sind, so dass sich beim Dichtsintern, wodurch dann das Zahnersatzteil erzeugt wird, eine Kontraktion des Materials in allen Raumrichtungen der gleichen Größe ergibt.

Das Dichtsintern erfolgt z. B. bei Temperaturen zwischen 1200 °C und 1600 °C, wie etwa bei 1400 °C über einen Zeitraum von 10 bis 16 Stunden. Bevorzugt ist ein Dichtsintern bei 1400 °C für 14 Stunden.

Das Zahnersatzteil besteht aus einem der oben oder weiter unten genannten Materialien.

Die Erfindung betrifft weiterhin Material für einen Rohling zum Herstellen von Zahnersatz. Das Material wird durch Dichtsintern in ein Material für Zahnersatz nach einem der Ansprüche 1-5 und wie oben und weiter unten beschrieben, umgewandelt. Dieses Rohlingsmaterial hat eine Biegefestigkeit von mindestens 20 und höchstens 180 MPa und sämtliche Zwischenwerte oder Zwischenintervalle sind möglich (siehe auch Anspruch 9 und 10). Je geringer diese 3-Punkt-Biegefestigkeit ist, desto leichter lässt sich das Material beispielsweise durch Fräsen und dabei ohne hohen Fräswerkzeugverschleiß bearbeiten. Je höher die Biegefestigkeit ist, desto höhere Belastungen hält das Material beim Fräsen aus, so dass ein schnelleres Fräsen möglich wird. In dem genannten Intervall ist ein guter Kompromiss zwischen den verschiedenen Anforderungen gegeben.

Die chemische Zusammensetzung dieses Rohlings-Materials entspricht der Zusammensetzung des Materials für Zahnersatz, da sich beim Dichtsintern die chemische Komposition nicht ändert.

Ein Rohling zum Herstellen von Zahnersatz weist vorzugsweise ein oben genanntes Material für einen Rohling zum Herstellen von Zahnersatz auf.

Weiterhin ist ein Rohling zur Herstellung von Zahnersatz dadurch gekennzeichnet, dass das Material des Rohlings bei einem Pressdruck von mindestens 1500 bar isostatisch oder uniaxial oder biaxial oder triaxial gepresst wurde.

Es hat hierbei eine Dichte von 3,10 - 3,30 g/m³ vorzugsweise 3,15 - 3,25 g/cm³.

Es hat sich gezeigt, dass durch das Pressen mit einem sehr hohen Pressdruck das Rohlingsmaterial nach dem Dichtsintern eine sehr hohe 3-Punkt-Biegefestigkeit aufweist. Auch wenn sich die Dichte nach dem Dichtsintern praktisch unabhängig von dem Rohlingspressdruck zeigt, so ergibt sich überraschenderweise hier aber doch eine Verbesserung der 3-Punkt-Biegefestigkeit aufgrund eines hohen Pressdrucks.

Die Dicke des dichtgesinterten Materials beträgt vorzugsweise 6,00 bis 6,10 oder 6,05 bis 6,07 g/cm³.

Insbesondere hat sich hierbei gezeigt, dass sich die 3-Punkt-Biegefestigkeit von einem größeren Rohlingskörper, aus dem mehrere Rohlinge gewonnen werden können, sehr zuverlässig für sämtliche Rohlinge des Rohlingskörpers ergibt. Die Variation der 3-Punkt-Biegefestigkeit innerhalb eines derart hergestellten Rohlingskörpers ist relativ gering.

Der Pressdruck kann auch höher als 1500 bar sein, d. h. bis zu 3000 oder 4000 bar (s. a. Anspruch 13 und 14). Das Pressen des Materials zum Herstellen des Rohlings kann bei Umgebungstemperatur oder auch erhöhter Temperatur stattfinden.

Der Rohling hat vorzugsweise eine Scheibenform, wobei sich hierbei eine kreisrunde, quadratische, rechteckige oder polygonale Scheibenform als vorteilhaft herausgestellt hat, für die weitere Bearbeitung zur Gewinnung von Zahnersatzteilen aus dem Rohling.

An der Scheibenseite kann eine Markierung entweder durch eine Nut oder eine farbige oder sonstartige Markierung vorgesehen sein, mit der die Orientierung des Rohlings eindeutig festlegbar ist.

Weiterhin ist es vorteilhaft, wenn die Scheibenseite keine Stufen aufweist, da sich dann eine einfache geometrische Form des Rohlings ergibt. Beim Herstellen von Zahnersatzteilen müssen die Zahnersatzteile in einer vergrößerten Form aus dem Rohling herausgearbeitet werden, da sich beim nachfolgenden Dichtsintern das Material kontrahiert. Der Vergrößerungsfaktor entspricht hierbei der dritten Wurzel aus dem Quotient der Zieldichte und der Vordichtsinterungsdichte. Dadurch, dass die Scheibenseite keine Stufen aufweist, lässt sich das Volumen der Scheibe möglichst exakt und einfach durch Abmessen der Größe des Rohlings bestimmen, so dass auch die Vorsinterungsdichte möglichst exakt und leicht bestimmt werden kann.

Wie bereits oben erwähnt, führt das Herstellen eines Rohlings zum Herstellen von Zahnersatz durch Pressen mit einem hohen Pressdruck zu einer guten Homogenität des Rohlingsmaterials. Deshalb ist im weiteren ein Satz von Rohlingen, insbesondere von Rohlingen aus ein und demselben Rohlingskörper gegeben, wobei die Rohlinge mittels Dichtsintern eine 3-Punkt-Biegefestigkeit von mindestens 1300 bis 2000 MPa aufweisen können.

Weiterhin ist ein Satz von Rohlingen aus ein und demselben Rohlingskörper dadurch gekennzeichnet, dass der Rohlingskörper bei einem Pressdruck von mindestens 1500 bar isostatisch, uniaxial, biaxial oder triaxial gepresst wurde.

Auch betrifft die Erfindung ein Verfahren zum Herstellen von Rohlingskörpern oder Rohlingen für die Herstellung von Zahnersatz. Bei den Verfahren wird ein Pulver mit einem isostatischen oder uniaxialen, biaxialen oder triaxialen Pressdruck von mindestens 1500 bar zu einem Rohlingskörper gepresst. Der Druck kann auch 4100 bar oder einen Zwischenwert zwischen 1500 und 4100 bar betragen oder in sämtlichen Zwischenintervallen zwischen 1500 und 4100 bar liegen (s. a. Anspruch 22 und 23).

Ein derartig hergestellter Rohlingskörper oder Rohling kann eine 3-Punkt-Biegefestigkeit im Bereich von zwischen 20 und 180 MPa oder sämtlichen Zwischenwerten oder aus möglichen Zwischenintervallen zwischen diesen Werten (s. a. Anspruch 9 und 10) aufweisen. Damit ist eine Bearbeitbarkeit des Rohlingsmaterials gegeben. Wird dieses Material dichtgesintert, so ergibt sich eine 3-Punkt-Biegefestigkeit von mindestens 1300 bis zu 2000 MPa.

Bei dem Verfahren wird das Pulver zur Pressung beispielsweise in einem gummi-elastischen Körper eingefüllt. Das Befüllen findet vorzugsweise in einem Sauberraum statt, so dass ungewünschte Verunreinigungen des Pulvers möglichst klein gehalten werden.

Das Pulver kann ein Oxid mit evtl. einer Metallsalzbeimischung sein, wie sie weiter oben angegeben worden ist.

Das Pressen findet vorzugsweise in einer hydrostatischen Presse statt, mit der ein isostatisches Pressen des Pulvers möglich wird. Ein derartig gepresstes Pulver führt zu einem Rohling mit einer sehr homogenen Dichte einer guten Materialkontraktion beim Dichtsintern in der Hinsicht, dass die Kontraktion in verschiedenen Raumrichtungen gleich groß ist. Durch das Pressen ergibt sich ein Rohlingskörper, der in der Regel nicht völlig regelmäßig geformt ist. Insbesondere beim isostatischen Pressen ergibt sich ein Rohlingskörper mit einer gewellten Oberfläche. Es ist daher vorteilhaft, einen solchen Rohlingskörper nach dem Pressen zu überdrehen oder plan zu bearbeiten. Das Überdrehen führt zu einer kreiszylindrischen Form. Beim Planbearbeiten werden Oberflächen des Rohlingskörpers so bearbeitet, dass sie anschließend eben oder plan sind. Dies führt beispielsweise zu Rohlingskörpern mit einem quadratischen oder rechteckigen Querschnitt, wenn die verschiedenen hergestellten Ebenen senkrecht zueinander sind oder zu einem sonstigen polygonalen Querschnitt.

Bei dem Verfahren wird der Rohlingskörper vorzugsweise in mehrere Rohlinge zerlegt. Dies geschieht vorzugsweise durch Trennen entlang von Ebenen, die vorzugsweise parallel zueinander und in etwa senkrecht zu einer Längsachse des Rohlingskörpers liegen, mit dem Verfahren ist es jedoch auch möglich, unmittelbar Rohlinge herzustellen und nicht erst Rohlingskörper, die dann zertrennt werden. So können beispielsweise auch bei einem einzelnen Pressvorgang mehrere Rohlinge separat hergestellt werden. Auch können bei einem einzelnen Pressvorgang mehrere Rohlingskörper separat hergestellt werden, jeder in einem separaten gummi-elastischen Körper.

Vorteilhaft ist weiterhin ein solches Verfahren, bei dem der Rohlingskörper längs einer Längsachse dieses Rohlingskörpers mit einer Markierung versehen wird. Die Markierung kann eine Nut oder eine farbige oder sonst wie ausgestaltete Linie sein. Auch Bar-Codes oder ähnliches können hier auf den Rohlingskörper aufgebracht werden.

Die Erfindung betrifft auch ein Verfahren zum Herstellen eines Zahnersatzteils unter Verwendung eines der beschriebenen Rohlinge oder eines nach dem beschriebenen Verfahren hergestellten Rohlings. Die Bearbeitung ist vorzugsweise Fräsen.

Vorteilhafte Ausführungsformen der Erfindung sollen anhand von den beiden Figuren erläutert werden. Dabei zeigt:
Fig. 1 eine schematische Schnittansicht einer isostatischen Presse,
Fig. 2 dreidimensionale schematische Ansichten von Rohlingskörpern,
Fig. 3 eine dreidimensionale schematische Ansicht von einem Rohlingskörper und daraus gewonnenen Rohlingen,
Fig. 4 eine dreidimensionale schematische Ansicht eines Rohlings.

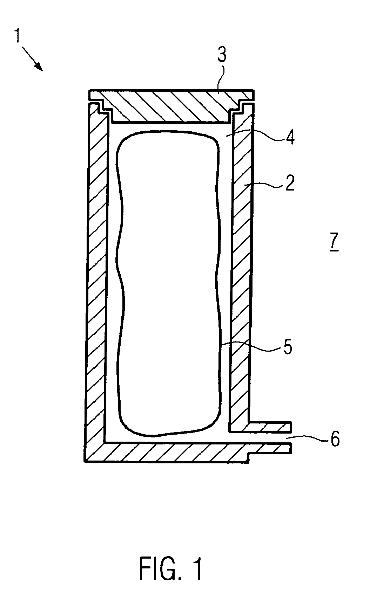

In Fig. 1 ist eine hydrostatische Presse 1 gezeigt, in die ein gummielastischer Körper 5 eingesetzt ist, der seinerseits mit einem Pulver wie beispielsweise einem Zirkonoxidpulver und Zusätzen gefüllt ist.

Der gummielastische Körper 5 befindet sich in einem Innenraum 4, der durch einen Behälter 2 und einen Deckel 3 geschaffen wird. Durch eine Zuleitung 6 kann hydrostatischer Druck auf dem gummielastischen Körper 5 und somit auf das im gummielastischen Körper 5 befindlichen Pulver ausgeübt werden.

Der gummielastische Körper 5 wurde in einem Sauberraum 7 mit dem Pulver gefüllt und verschlossen.

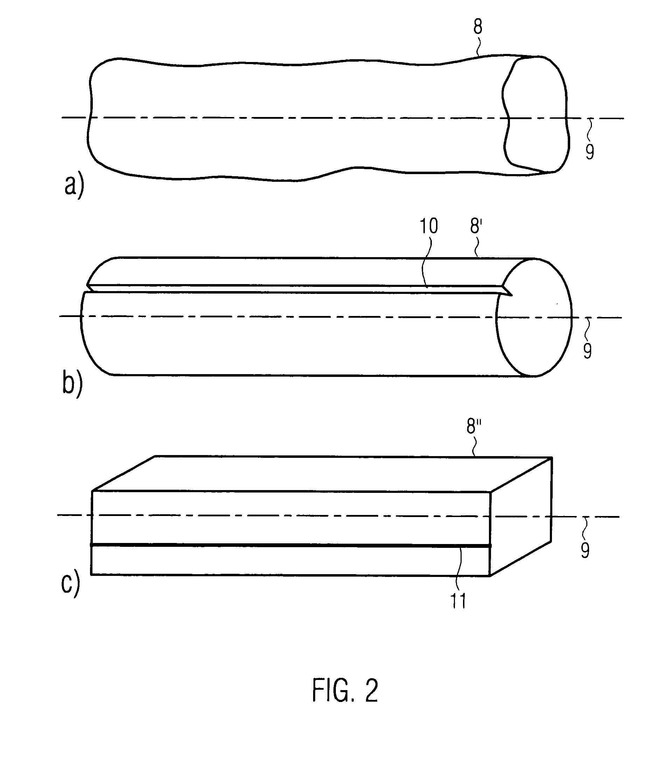

Ein mit der hydrostatischen Presse aus Fig. 1 gewonnener Rohlingskörper ist in Fig. 2a schematisch dargestellt. Der Rohlingskörper 8 weist eine Längsachse 9 auf sowie weiterhin eine etwas unregelmäßig gewellte Oberfläche. Durch Überdrehen des Rohlings 8 kann dieser in einen rotationssymmetrischen Rohlingskörper 8' (siehe Fig. 2b) umgearbeitet werden. In diesem Rohlingskörper 8' kann auch eine Nut 10 vorgesehen werden, mit der Rohlinge in ihrer Lage nach dem Zerteilen des Rohlingskörpers 8' zugeordnet oder erkannt werden. Hierfür ist/sind insbesondere eine Nut mit einem asymmetrischen Querschnitt oder mehrere asymmetrisch angeordnete Nuten vorteilhaft.

In Fig. 2c ist eine bevorzugte Ausführungsform eines solchen Rohlingskörpers 8" gezeigt, der plan bearbeitet wurde, so dass sich ein quadratischer oder rechteckiger Querschnitt ergibt. Der Rohlingskörper wird mit einer Nut oder Markierung 11 versehen, die vorzugsweise derart angeordnet ist, dass sie nicht auf einer Symmetrie-Ebene oder Achse des Rohlingskörpers 2a liegt, so dass eine eindeutige Lageerkennung des Rohlings, der aus diesem Rohlingskörper 8" gewonnen wird, möglich ist. Auch hier sind asymmetrisch und/oder mehrere asymmetrisch angeordnete Nuten vorteilhaft.

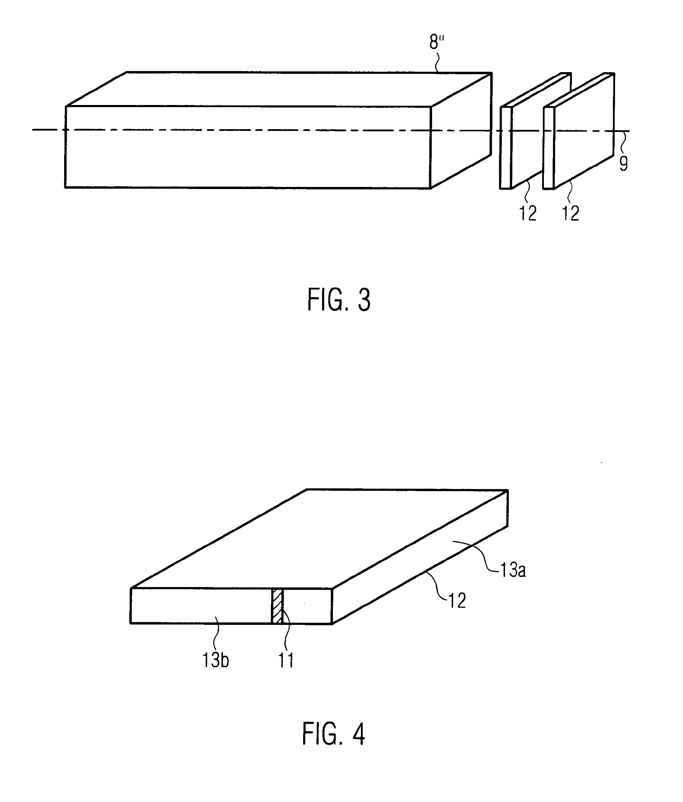

In Fig. 3 ist beispielhaft gezeigt, wie der Rohling 8" durch Trennen entlang von Ebenen senkrecht zu der Achse 9 in einzelne Rohlinge 12 zerteilt wird.

Statt erst Rohlingskörper 8 zu gewinnen, die anschießend zerteilt werden, ist es jedoch auch möglich, unmittelbar Rohlinge zu pressen. Das Zertrennen in Rohlinge kann dann entfallen. Die Oberflächen von durch Pressen gewonnenen Rohlingen werden in der Regel auch leicht uneben sein, so dass sich hier eine Planbearbeitung dieser Oberflächen anbietet, um Rohlinge mit einfachen geometrischen Formen zu erhalten.

In Fig. 4 ist schematisch ein Rohling 12 dargestellt. Dieser Rohling hat eine Scheibenform mit Scheibenseiten 13a und 13b.

Die Seiten 13a und 13b sind stufenlos, d. h. in der Richtung von der oberen zur unteren Seite ist die Seite ohne Stufen. In einer Richtung senkrecht dazu können Stufen z. B. durch eine Nut gegeben sein.

Auf der Seite 13b ist weiterhin eine Markierung zu erkennen, die der Markierung 11 aus der Fig. 2c entspricht. Da diese Markierung nicht mittig auf der Seite 13b angebracht ist (was jedoch auch möglich wäre), kann durch das Finden der Markierung 11 die Lage des Rohlings 12 eindeutig zugeordnet werden. Wird der Rohling beispielsweise um 180° gedreht und zwar um eine Achse, die in der Scheibenebene liegt und durch die Fläche 13b hindurchgeht, so befindet sich die Markierung 11 weiter links statt, wie die Fig. 4, weiter rechts von der Mitte. Weiterhin wird durch die Markierung 11 eine der vier Scheibenseiten ausgezeichnet, so dass sich mit der Markierung 11 die Lage des Rohlings 12 eindeutig bestimmen lässt.

Ein solcher Rohling, wie er in Fig. 4 dargestellt ist, weist eine 3-Punkt-Biegefestigkeit zwischen 20 und 180 MPa auf. Nach dem Dichtsintern eines solchen Rohlings- bzw. von einem aus diesem Rohling gewonnenen Zahnersatzteil beispielsweise durch Fräsen, ergibt sich ein Material mit einer 3-Punkt-Biegefestigkeit von mindestens 1300 MPa.

Hier sind auch Werte von bis zu über 1500 oder auch höher als 1600 und 1700, 1800 oder sogar über 1900 MPa möglich.

## Patentansprüche

1. Material für Zahnersatz
**gekennzeichnet durch**
eine 3-Punkt Biegefestigkeit von mindestens 1300 MPa.

2. Material nach Anspruch 1, **dadurch gekennzeichnet, dass** das Material ein keramisches Material, wie etwa Zirkonoxid, ist oder umfasst.

3. Material nach einem der Ansprüche 1 oder 2, **dadurch gekennzeichnet, dass** die 3-Punkt Biegefestigkeit mindestens 1400, 1500, 1600, 1700, 1800, 1900 oder 2000 MPa beträgt.

4. Material nach einem der Ansprüche 1 bis 3, **dadurch gekennzeichnet, dass** es Dichteschwankungen von weniger als 10 %, 5 % oder 2 % aufweist.

5. Material nach einem der Ansprüche 1 bis 4, **dadurch gekennzeichnet, dass** das Material umfasst:
90 % bis 99 % Zirkonoxid,
0 % bis 4 % Hafniumoxid,
1,5 % bis 6 % Yttriumoxid, und
0,01 % bis 0,75% von einem oder mehreren der Oxide von Aluminium, Gallium, Germanium, Indium, Zinn, Blei, Lanthanide, Metall, Eisen und/oder von einem oder mehreren Metallsalzen.

6. Zahnersatzteil umfassend oder bestehend aus einem Material nach einem der Ansprüche 1 bis 5.

7. Material für einen Rohling zum Herstellen von Zahnersatz, das durch Dichtsintern in ein Material für Zahnersatz nach einem der Ansprüche 1 bis 5 umwandelbar ist.

8. Material für einen Rohling zum Herstellen von Zahnersatz, das durch einen isostatischen, uniaxialen, biaxialen oder triaxialen Pressdruck von mindestens 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900 oder 4000 bar und/oder maximal nicht mehr als 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000, oder 4100 bar erhaltbar ist.

9. Material nach Anspruch 7 oder 8, **dadurch gekennzeichnet, dass** es eine 3-Punkt Biegefestigkeit von mindestens 20, 30, 40, 50, 60, 70, 80, 90, 100, 110, 120, 130, 140, 150, 160 MPa aufweist.

10. Material nach einem der Ansprüche 7 bis 9, **dadurch gekennzeichnet, dass** es eine 3-Punkt Biegefestigkeit von höchstens 30, 40, 50, 60, 70, 80, 90, 100, 110, 120, 130, 140, 150, 160, 170, 180 MPa aufweist.

11. Material nach einem der Ansprüche 7 bis 10, **dadurch gekennzeichnet, dass** das Material umfasst:
90 % bis 99 % Zirkonoxid,
0 % bis 4 % Hafniumoxid,
1,5 % bis 6 % Yttriumoxid, und
0,01 % bis 0,75% von einem oder mehreren der Oxide von Aluminium, Gallium, Germanium, Indium, Zinn, Blei, Lanthanide, Metall, Eisen und/oder von einem oder mehreren Metallsalzen.

12. Rohling (12) zur Herstellung von Zahnersatz umfassend ein Material der Ansprüche 7 bis 11.

13. Rohling (12) zur Herstellung von Zahnersatz,
**dadurch gekennzeichnet, dass**
der Rohling erhaltbar ist durch isostatisches oder uniaxiales, biaxiales oder triaxiales Pressen bei einem Pressdruck von mindestens 1500 bar.

14. Rohling nach Anspruch 13, **dadurch gekennzeichnet, dass** der Pressdruck mindestens 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900 oder 4000 bar beträgt.

15. Rohling nach Anspruch 13 oder 14, **dadurch gekennzeichnet, dass** der Pressdruck nicht mehr als 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000, oder 4100 bar beträgt.

16. Rohling nach einem der Ansprüche 12 bis 15, **dadurch gekennzeichnet, dass** der Rohling (12) eine Scheibenform hat, wobei der Rohling (12) vorzugsweise kreisrund, quadratisch, rechteckig oder polygon ist.

17. Rohling nach Anspruch 16, **dadurch gekennzeichnet, dass** der Rohling (12) an der Scheibenseite (13b) ein Markierung (11) wie etwa eine Nut und/oder eine farbige Linie und/oder einen Barcode aufweist.

18. Rohling nach Anspruch 16 oder 17, **dadurch gekennzeichnet, dass** die Scheibenseite (13a, 13b) stufenlos ist.

19. Satz von Rohlingen insbesondere von Rohlingen aus ein und demselben Rohlingskörper, **dadurch gekennzeichnet, dass** alle Rohlinge des Satzes durch Dichtsintern so umwandelbar sind, dass sie eine 3-Punkt Biegefestigkeit von mindestens 1300 , 1400, 1500, 1600, 1700, 1800, 1900 oder 2000 MPa aufweisen.

20. Satz von Rohlingen aus ein und demselben Rohlingskörper, **dadurch gekennzeichnet, dass** der Rohlingskörper durch Pressen bei einem isostatischen, uniaxialen, biaxialen oder triaxialen Pressdruck von mindestens 1500, 1600, 1700, 1800, 1900, 2000, 2100, 2200, 2300, 2400, 2500, 2600, 2700, 2800, 2900, 3000, 3,100, 3200, 3300, 3400, 3500, 3600, 3700, 3800, 3900 oder 4000 bar und/oder maximal nicht mehr als 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000, oder 4100 bar erhaltbar ist.

21. Verfahren zur Herstellung von Rohlingskörpern (8) oder Rohlingen (12) für die Herstellung von Zahnersatz,
bei dem ein Pulver mit einem isostatischen oder uniaxialen, biaxial, oder triaxialen Pressdruck von mindestens 1500 bar zu einem Rohlingskörper gepresst wird.

22. Verfahren nach Anspruch 21, **dadurch gekennzeichnet, dass** der Pressdruck mindestens 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900 oder 4000 bar beträgt.

23. Verfahren nach Anspruch 21 oder 22, **dadurch gekennzeichnet, dass** der Pressdruck nicht mehr als 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000 oder 4100 bar beträgt.

24. Verfahren nach einem der Ansprüche 21 bis 23, **dadurch gekennzeichnet, dass** das Pulver zur Pressung in einen gummielastischen Körper (5) eingefüllt wird.

25. Verfahren nach Anspruch 24, **dadurch gekennzeichnet, dass** das Befüllen in einem Sauberraum (7) vorgenommen wird.

26. Verfahren nach einem der Ansprüche 21 bis 25, **dadurch gekennzeichnet, dass** das Pressen mit einer hydrostatischen Presse (1) durchgeführt wird.

27. Verfahren nach einem der Ansprüche 21 bis 26, **dadurch gekennzeichnet, dass** der Rohlingskörper (8) oder der Rohling (12) nach dem Pressen überdreht oder planbearbeitet wird.

28. Verfahren nach einem der Ansprüche 21 bis 27, **dadurch gekennzeichnet, dass** der Rohlingskörper (8) in mehrere Rohlinge (12) zerlegt wird, wobei dies vorzugsweise durch Trennen entlang einer Ebene geschieht, die vorzugsweise in etwa senkrecht zu einer Längsachse (9) des Rohlingskörpers (8) liegt.

29. Verfahren nach einem der Ansprüche 21 bis 28, **dadurch gekennzeichnet, dass** die Rohlinge (12) eine Scheibenform haben, wobei die Scheibe vorzugsweise kreisförmig, quadratisch, rechteckig oder polygonal ist.

30. Verfahren nach einem der Ansprüche 21 bis 29, **dadurch gekennzeichnet, dass** der Rohlingskörper (8) längs einer Längsachse (9) des Rohlingskörpers (8) mit einer Markierung (11) wie einer Nut (10) und/oder farbigen Linie (11) und/oder einem Barcode versehen wird.

31. Verfahren zum Herstellen von einem Zahnersatzteil oder von Zahnersatzteilen mit den Schritten:
a) - Herstellen von Rohlingen nach einem der Verfahren der Ansprüche 21 bis 30 oder Herstellen von Rohlingskörpern und dem anschließenden Gewinnen von Rohlingen nach einem der Ansprüche 21 bis 30;
oder
- Bereistellen eines Rohlings nach einem der Ansprüche 12 bis 18; oder von einem Satz von Rohlingen nach einem der Ansprüche 19 oder 20;
b) Bearbeiten des Rohlings oder der Rohlinge des Rohlingssatzes, um ein Zahnersatzteil oder mehrere Zahnersatzeile zu formen, beispielsweise durch Fräsen.

32. Verfahren nach Anspruch 30, **dadurch gekennzeichnet, dass** der Rohling ein keramisches Material umfasst, dass dichtgesintert wird, vorzugsweise bei einer Temperatur zwischen 1200 °C und 1700 °C, 1300 °C und 1650 °C oder 1350 °C und 1600 °C, wie etwa bei 1400 ± 10 oder 20 °C und zwar für eine Zeit von 10 Stunden bis 16 Stunden, bevorzugt 14 Stunden.
